(19) **Europäisches Patentamt / European Patent Office / Office européen des brevets**

(11) **EP 3 013 210 B1**

(12) **EUROPEAN PATENT SPECIFICATION**

(45) Date of publication and mention
of the grant of the patent:
**13.03.2024 Bulletin 2024/11**

(21) Application number: **14761958.9**

(22) Date of filing: **26.06.2014**

(51) International Patent Classification (IPC):
**A61B 3/00** (2006.01)

(52) Cooperative Patent Classification (CPC):
**A61B 3/0025; G06T 7/0016;** G06T 2207/30041

(86) International application number:
**PCT/EP2014/063619**

(87) International publication number:
**WO 2014/207161 (31.12.2014 Gazette 2014/53)**

(54) **CHANGE ANALYSIS SYSTEM & METHOD**

ÄNDERUNGSANALYSESYSTEM UND -VERFAHREN

SYSTÈME ET PROCÉDÉ D'ANALYSE DE CHANGEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.06.2013 GB 201311310**

(43) Date of publication of application:
**04.05.2016 Bulletin 2016/18**

(73) Proprietor: **The City University**
**London EC1V 0HB (GB)**

(72) Inventors:
• **ZHU, Haogang**
**London EC1V 0HB (GB)**
• **CRABB, David**
**London EC1V 0HB (GB)**

• **GARWAY-HEATH, David**
**London N6 5EX (GB)**

(74) Representative: **Milhench, Mark Lorne**
**First Thought IP Ltd**
**The Ramblers**
**Gents Lane, Shimpling**
**Bury St. Edmunds, Suffolk IP29 4HP (GB)**

(56) References cited:
**US-A1- 2011 287 801**

• **DAVID P. CRABB ET AL: "Improving the
Prediction of Visual Field Progression in
Glaucoma Using Spatial Processing",
OPHTHALMOLOGY, vol. 104, no. 3, 1 March 1997
(1997-03-01) , pages 517-524, XP055151035,
ISSN: 0161-6420, DOI:
10.1016/S0161-6420(97)30281-4**

**Description**

Field

This invention relates, in general, to change analysis systems and methods in the context of retinal analysis.

Background

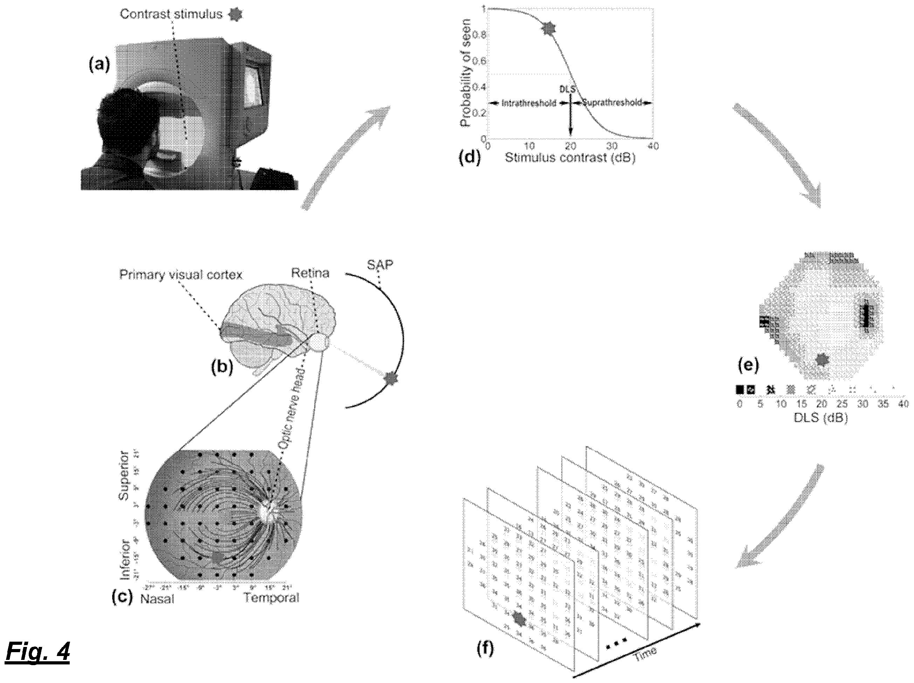

In recent years giant strides have been made in understanding of the biology of ocular disease in the research laboratory and in vivo, leading to the elucidation of neuro-regenerative processes and even reversing blindness in some conditions. The retina, uniquely, is an accessible and directly visible extension of the brain and, therefore, retinal research is becoming a focus for unravelling the complexity of other neurological changes such as those observed in Alzheimer's disease, multiple sclerosis and Gaucher disease. The primary endpoint in eye-related research or therapy is preservation or improvement in visual function. The established reference test for a fundamental aspect of visual function, namely the visual field, is Standard Automated Perimetry (SAP; Fig. 4(a)). SAP measures the differential light sensitivity (DLS), across a person's retina (Fig. 4(c)) and the corresponding visual pathway (Fig. 4(b)). Unfortunately, development of computational and statistical methods for analysing data from SAP has not kept pace with the advances in the biology of eye related research.

SAP is used extensively in eye and neurology clinics. A major application is in the detection and management of glaucoma, a group of chronic optic neuropathies causing progressive loss of retinal ganglion cells and their axons and resulting in loss of retinal function. This disease represents a large global health problem with about 80 million people by expected to be affected by 2020. Computational methods are required to analyse series of SAP data; without these, even experienced clinicians have been shown to make inconsistent decisions. Current statistical approaches typically use ordinary least squares regression over time to track changes in summary measures, regions of interest or individual visual field locations. Other methods simply make comparisons between the most recent test(s) and a baseline measurement.

DAVID P. CRABB ET AL: "Improving the Prediction of Visual Field Progression in Glaucoma Using Spatial Processing",OPHTHALMOLOGY, vol. 104, no. 3, 1 March 1997 (1997-03-01), pages 517-524, discloses a prediction based on error model and spatial correlation model based on historical data.

Current methods for detecting change in a series of measurements from SAP over time are inadequate because they do not sufficiently address the complexity of the data yielded from the test. This means that detection of change in retinal function is often delayed, or requires many clinical visits. Data from SAP are complex and have difficult statistical properties, particularly non-stationary variability and spatial correlation.

Firstly, SAP does not directly measure retinal function; measurements are indirect because of the psychophysical process where a person's response depends on the probability of perceiving and responding to a light stimulus (Fig. 4(d)) Moreover, measurement variability abounds and differs considerably from patient to patient. The measurement variability is non-stationary and worsens as DLS deteriorates with disease, eventually becoming censored in blind regions.

Secondly, SAP measurements tend to be made in a regular grid across a patient's field of view. This regular grid does not respect the anatomical arrangement of the retinal nerve fibres that transmit signals from the retina to the brain (Fig. 4(c)). The division of the grid by retinal nerve fibres results in correlation between spatially-related locations. There are prescriptions for modelling this unique spatial process, but they have yet to be incorporated into analysis of series of SAP measurements over time.

Aspects of the present invention seek to address one or more of the drawbacks associated with conventional approaches to change analysis.

Summary

To this end, a presently preferred implementation of the teachings of the invention provides a change analysis system which is defined in claim 1.

In the illustrative context of retinal analysis, this aspect of the present invention adopts a computational approach to the handling of difficult variability structure in SAP data, whilst also capturing information about the spatial process underpinning changes in the visual field. In one embodiment referenced generally herein as "Analysis with non-stationary *Weibull* error regression and spatial enhancement" (*ANSWERS*) it is possible to accurately identify changes in SAP measurements acquired over time. This notwithstanding, the teachings of the invention can readily be applied to investigations of new therapies, so that changes before and after an intervention can be detected. We have also determined that *ANSWERS* can detect change in retinal function more rapidly than conventional methods that are based on ordinary linear regression. Reference will also be made herein to a variation of *ANSWERS* that is not spatially enhanced, and named "ANSWER" (Analysis with non-stationary *Weibull* error regression).

In preferred embodiments, the reporting module may be operable to generate a report concerning the rate of change in said analysed dataset. The reporting module may, additionally or alternatively, be operable to generate a report concerning a probability of change in said analysed dataset. In this instance, the probability of change may comprise a probability of change having already occurred or a probability of a change occurring at some point in the future.

In one embodiment, the expected change module may be configured to generate said model of expected changes from changes in another set of data that is related to the dataset to be analysed. The other set of data may comprise data pertaining to a different type of test to a test or tests from which said dataset to be analysed resulted. In a preferred arrangement, one of said dataset to be analysed and said another set of data may comprise data from one or more functional tests that measure the function of an object, and the other of said dataset to be analysed and said another set of data may comprise data from one or more structural tests that measure the structure of an object. The structural test may generate data concerning the structure of a biological object. The functional test may generate data concerning the function of said biological object. In one example, the biological object comprises the retina of a subject.

In a preferred implementation, the error profiling module is operable to model heteroscedastic error. Alternatively or additionally, the error profiling module may be operable to model non-normal error. In one implementation the error profiling module may be operable to fit one or a mixture of Weibull distributions to said historical data set.

The errors may comprise variability in measurements of a measured object over time. The errors may comprise noise, said noise comprising differences between true measurements and recorded measurements.

In preferred implementations, the spatial correlation quantification module may be configurable for a particular condition. For example, the spatial correlation quantification module may be configurable for glaucoma or macular degeneration.

In a preferred arrangement, the time series dataset that is to be analysed may comprise a set of data output directly from a testing device. This arrangement is particularly advantageous as building the analysis system herein disclosed into a testing device, for example a device for conducting SAP, provides the possibility of "on the spot" analysis of subject data.

In a preferred arrangement said target function construction module is operable to implement a Bayesian framework. In a preferred arrangement said expected change module outputs prior probability distributions in a Bayesian framework.

In an envisaged implementation of the teachings of the invention, one or more of said error profiling module, said spatial correlation quantification module, said expected change module, said target function construction module, said optimisation module and said reporting module comprise software instructions executable by a processor.

Another aspect of the present invention relates to a testing device, such as a visual field perimeter or a retinal structure imaging device, comprising a system according to any preceding claim.

A further aspect of the present invention relates to a change analysis method as defined in claim 13.

Another aspect of the present invention relates to a computer program which is defined in claim 15.

Other feature, advantages and embodiments of the present invention are set out below.

Brief Description of the Drawings

Various aspects of the teachings of the invention, and arrangements embodying those teachings, will hereafter be described by way of illustrative example with reference to the accompanying drawings, in which:

Fig. 1 is a schematic representation of an illustrative computer;
Fig. 2 is a schematic representation of software for the computer of Fig. 1;
Fig. 3 is a diagrammatic representation of a change analysis system according to an illustrative embodiment of the present invention;
Fig. 4 shows various steps of a SAP visual field measurement process;
Fig. 5 are graphs depicting retest distributions for DLS at ObB, 5dB, 15dB and 25dB;
Fig. 6 depicts a spatial correlation $\Sigma$ between each location and all other locations in a visual field;
Fig. 7 depicts histograms of of retest differential light sensitivities at levels between 0dB and 35dB
Figs. 8(a) and (b) are graphs comparing *ANSWER* with ordinary linear regression;
Fig. 9 depicts the time to detect deterioration for linear regression of mean deviation, pointwise linear regression, *ANSWERS* and *ANSWER;*
Fig. 10 depicts the hit rates of linear regression of mean deviation (*MD),* point-wise linear regression (*PLR),* ANSWERS and *ANSWER* with series lengths (*length)* of 3, 5, 7 and 9;
Figs 11(a) to (d) relate to an example of a short visual field series;
Figs. 12(a) to (d) relates to an example of a visual field series with an improving mean deviation;
Figs. 13(a) to (d) relate to an example visual field; and
Fig. 14 is a graph of *I*- threshold as a function of false positive rate and length of series.

Detailed Description

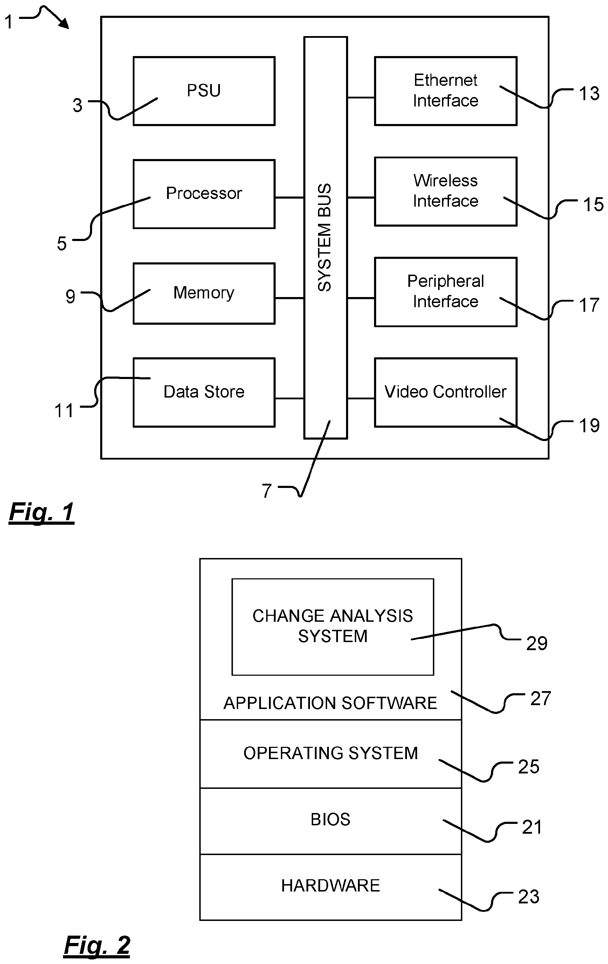

Fig. 1 is a schematic representation of an illustrative computer 1. The computer 1 comprises a power supply unit 3 that is configured to draw power from a mains power supply and regulate the supply of power to the remaining components of the computer.

The computer 1 includes a processor 5 that is coupled to a system bus 7 by means of which signals can be sent between the processor and the other components of the computer. The computer 1 further comprises read only memory (ROM) and/or random access memory (RAM) 9 that provides a processing environment in which the processor 5 can execute computer programs. The hub also includes a data store 11 for the storage of computer programs for execution by the processor, and data. The data store may comprise one or more hard disk drives (HDDs), and in a particularly preferred embodiment comprises a plurality of hard disk drives configured as a RAID (Redundant Array of Inexpensive Disks) to provide redundancy in the event that one or more drives of the array should fail.

In this illustrative embodiment, the system bus 7 is coupled to an Ethernet interface 13, a wireless interface 15, a peripheral interface 17 and a video controller 19. The Ethernet interface is configured to provide, a network connection between the computer 1 and other remote or local computers, for example by means of the world wide web. The wireless interface is configured to enable the computer to interface with a wireless network for the receipt and transmission of signals from and to the wireless network.

The peripheral interface 17 is configured to enable user interface devices, such as a keyboard and/or pointing device (such as a mouse or trackball), and ancillary equipment such as one or more printers to be connected to the computer for use therewith. The peripheral interface could include RS232 connectors, USB connectors, PS2 connectors or any other type of connector. The video controller 19 provides an interface that enables a display, not shown, to be coupled to the computer, and functions in response to signals from the processor to generate images for display on the display.

Fig. 2 is a schematic representation of software executable by the computer of Fig. 1. In the preferred arrangement the computer processor 5 and memory 9 cooperate to establish a BIOS (Basic Input/Output System) 21 that functions as an interface between the functional hardware components 23 of the computer and the software executed by the computer. The processor 5 then loads from memory 9 an operating system 25 which provides an environment in which application software 27 can run. In accordance with the preferred embodiment of the present invention the application software includes a change analysis system 29.

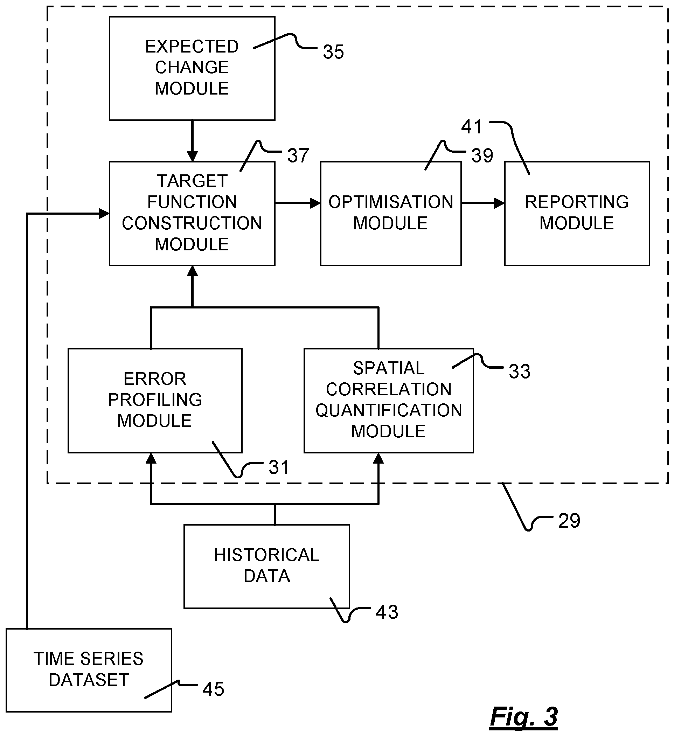

Fig. 3 is a more detailed depiction of the change analysis system 29. As shown, the system 29 comprises, in the preferred embodiment, a plurality of software modules that cooperate to provide the functionality hereafter described. In particular, the system includes an error profiling module 31, a spatial correlation quantification module 33, an expected change module 35, a target function construction module 37, an optimisation module 39 and a reporting module 41. As will hereafter be described in detail, historical data 43 is input to the error profiling module 31 and the spatial correlation quantification module, and a time series dataset 45 to be analysed is input into the target function construction module. The historical data 43, in this particular example, comprises time series data (that is to say, data concerning an individual patient that has been captured from measurements taken over a period of time) concerning a plurality of patients. The time series dataset 45 comprises time series data for a particular patient that is to be analysed by the system 29.

Referring now generally to the drawings, Fig. 4 depicts a visual field measured by static automated perimetry (SAP). In particular, Fig. 4(a) shows contrast stimuli from SAP being projected on different locations of a retina. The response from subject is captured when the stimulus is perceived. Fig. 4(b) illustrates how SAP assesses differential light sensitivity (DLS) of the retina and corresponding visual pathway. Fig. 4(c) depicts the measurement of DLSs at various locations (dots) on the retina. The point (0°,0°) indicates central vision that corresponds to the fovea on the retina. Optic nerve head is the anatomical blind spot. The test locations are not only correlated to their neighbours but also by the optic nerve fibres (some of which are shown as blue curves) passing through them. The whole visual field can be divided into superior and inferior hemifields on vertical and nasal and temporal regions on horizontal. In Fig. 4(d) the DLS at a location on the retina is derived at the 50% probability of the visual system responding to a contrast stimulus and is related to the biological response to light of relay neurones in the visual pathway. In Fig. 4(e) the DLS is measured in log scale between 0dB (high contrast stimulus, blindness) and round 35dB (low contrast stimulus, healthy) and is displayed as a conventional gray-scale plot. Darker shading represents lower DLS. Fig. 4(f) shows how the measurement of DLS over time forms a complex spatial-temporal time series.

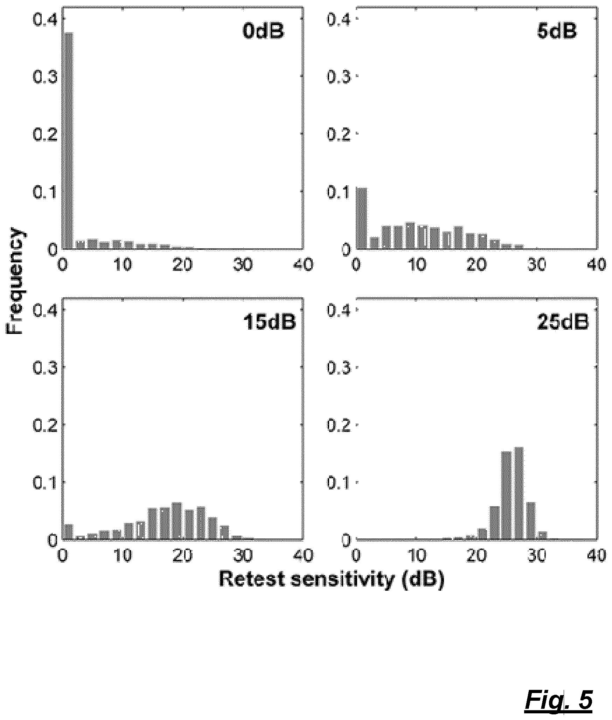

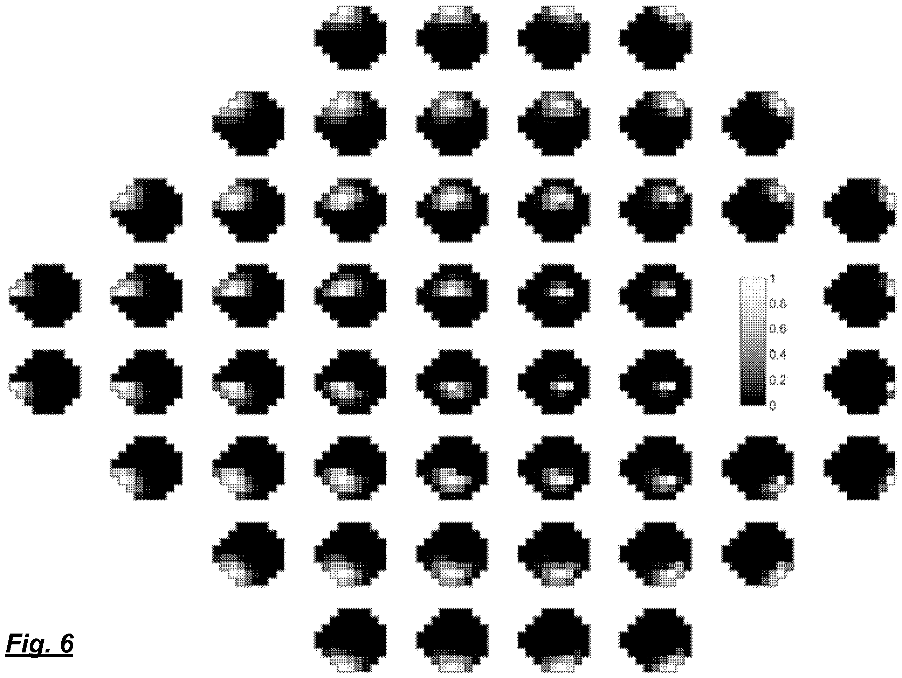

Fig. 5 shows retest distributions for differential light sensitivities at 0dB, 5dB, 15dB and 25dB. Fig. 6 shows the spatial correlation $\Sigma$ between each location and all other locations in the visual field. The composition of the graph is a 24-2 visual field as shown in Fig 4 (c). At each visual field location, an image, with the shape of a 24-2 visual field, represents the correlation between this location and all locations in the visual field. The gray scale bar, shown at the location of the blind spot, indicates the level of correlation

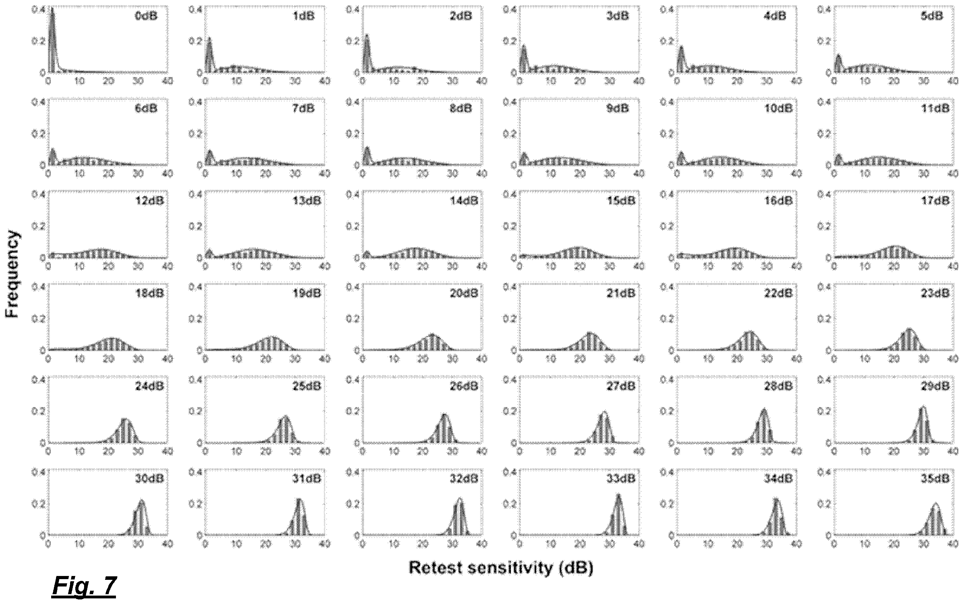

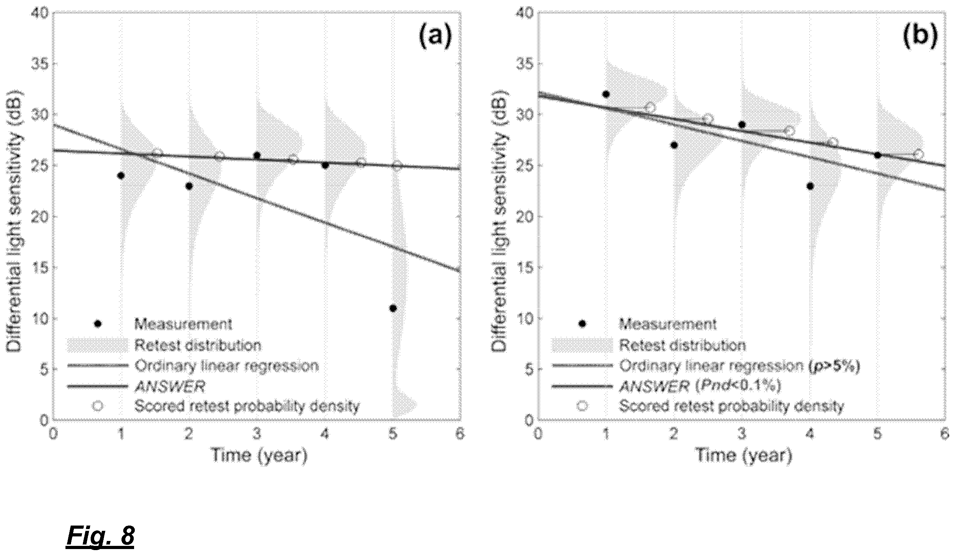

Fig. 7 shows histograms of retest differential light sensitivities at levels between 0dB and 35dB. The derived probability density function of the Weibull mixture is superimposed in red. Fig. 8 depicts charts comparing ANSWER and ordinary linear regression. The retest distributions of corresponding differential light sensitivity measurements are superimposed as gray areas and the scored probability densities by the ANSWER regression line are marked on the retest distributions.

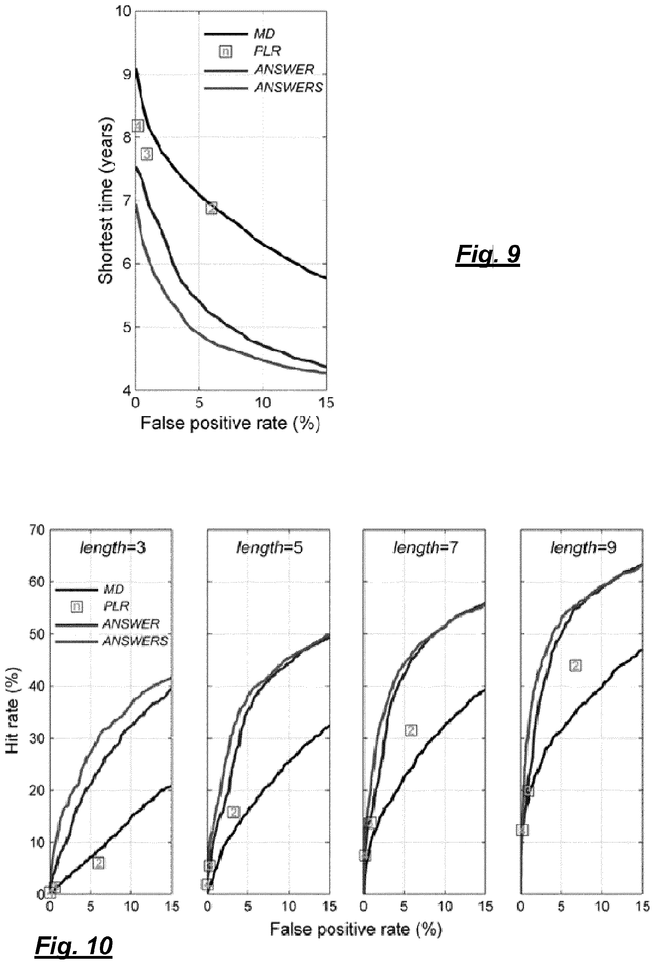

Fig. 9 is a chart of time to detect deterioration for linear regression of mean deviation (MD), point-wise linear regression (PLR), ANSWERS and ANSWER at false positive rates between 0 and 15%. The number of contiguous points in point-wise linear regression are shown in the square points. The lines/points on the graph correspond to the average time needed to first detect deterioration in series from clinical practice at given false positive rates.

Fig. 10 depicts the hit rates of linear regression of mean deviation (MD), point-wise linear regression (PLR), ANSWERS and ANSWER with series lengths (length) of 3, 5, 7 and 9. The number of contiguous points in point-wise linear regression are shown in the square points. The hit rates are estimated at false positive rates between 0 and 15%.

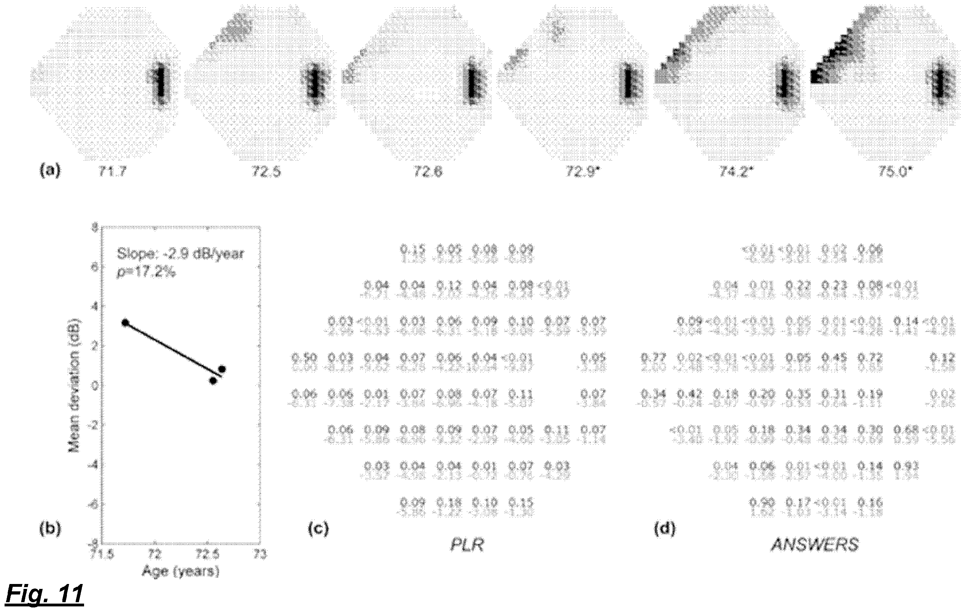

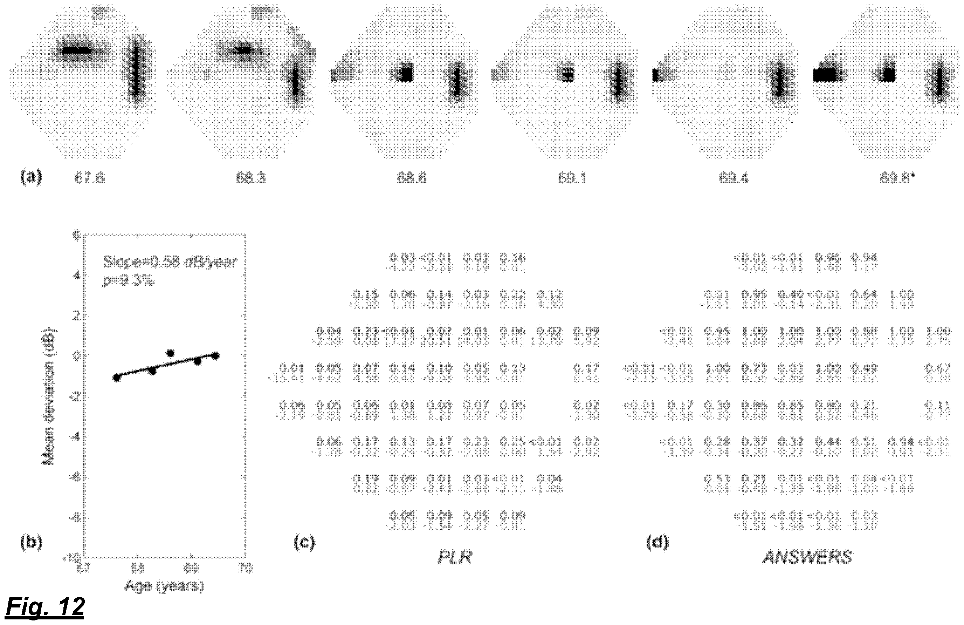

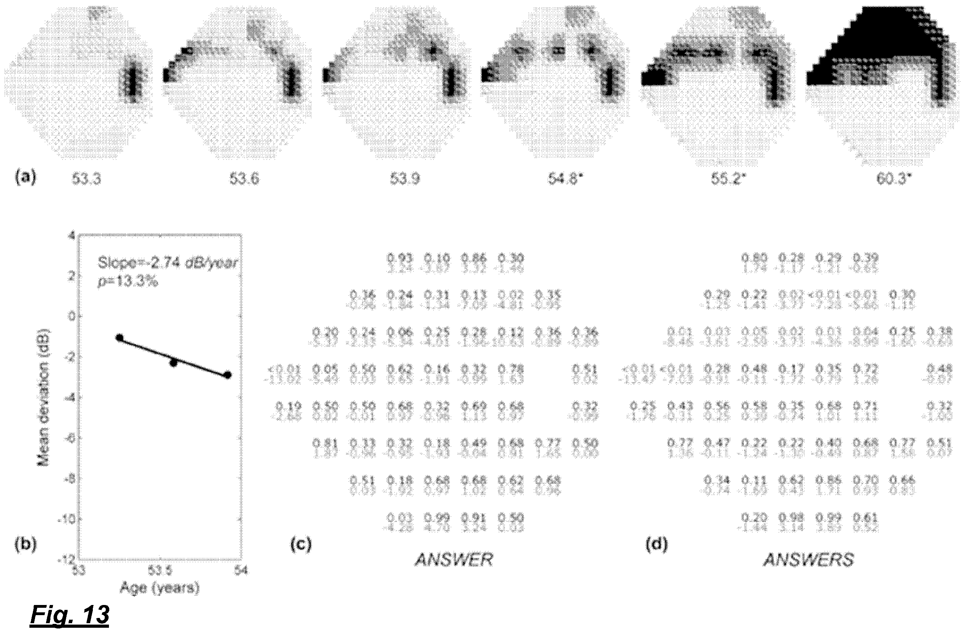

Figs. 11(a) to (d) relate to an example of short visual field series. Fig. 11(a) is a visual field series in conventional grey scale. The patient's age is shown under each visual field. The visual fields with stars after their age are shown to confirm deterioration status and were not included in the analysis. Fig. 11(b) shows linear regression of mean deviation. Fig. 11(c) shows the p-values and slopes in point-wise linear regression (PLR). The p-values for the location meeting the point-wise deterioration criteria (negative slope and p-value<1%) are coloured in red. Fig. 11(d) shows the Pnd values and slopes of ANSWERS. For visualisation purpose, the Pnd values <5% are coloured in red. Figs. 12(a) to (d) are related to an example visual field series with an improving mean deviation. The figure is arranged in the same configuration of Figs. 11(a) to (d). Figs. 13(a) to (d) relate to an example visual field series showing the usefulness of spatial enhancement in ANSWERS. Fig. 13(a) shows a visual field series in conventional grey scale. The patient's age is shown under each visual field. The last visual field is shown to confirm deterioration status and was not included in the analysis. Fig. 13(b) is a linear regression of mean deviation. Fig. 13(c) shows the Pnd values and slopes of ANSWERS without spatial enhancement (ANSWER). The Pnd values <5% are coloured in red. Fig. 13(d) shows the Pnd values and slopes of ANSWERS. The Pnd values <5% are coloured in red.

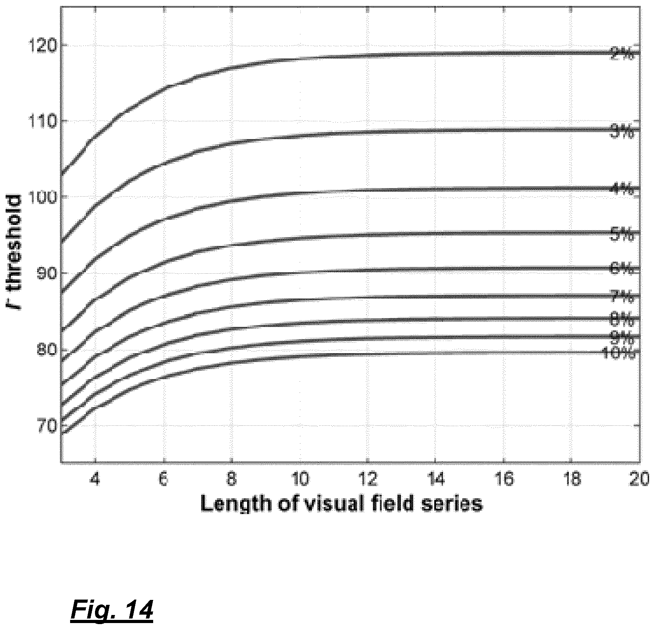

Fig. 14 depicts the $\Gamma$ threshold as a function of false positive rate and length of series (number of fields in the series). The $\Gamma$ threshold was estimated with false positive rates between 2% and 10% and length of series between 3 and 20.

The functionality embodied by each of the software modules shown in Fig. 3 will now be described in detail, and with particular reference to the mathematical functionality implemented by the modules.

The Error Profiling Module 31 uses probability density functions to establish the error (in particular, variability and noise) in the historical data. In a preferred arrangement, one or a mixture of *Weibull* distributions are employed, but it will be apparent to persons of ordinary skill in the art that other parametric (for example, a mixture of normal distributions) or non-parametric (for example, a kernel density estimator) density functions could instead be employed.

The variability of individual differential light sensitivity (DLS) measurements can be estimated by repeating visual field tests in a short period of time. In this particular instance, a test-retest dataset consisting of 1980 ($30C_{12}^{2}$ i.e., 30 multiplied by 12-choose-2 combinations) pairs of repeated visual field tests was used to estimate the retest distributions for DLS measurements ranging from 0dB to 35dB. As Fig. 5 shows, retest distributions are generally bimodal, truncated and skewed; the shape of the distribution varies dramatically across the range of DLS measurements due to the non-stationary variability and the censored nature of the DLS measurement. As the retest distribution cannot be sufficiently described by a single parametric probability density function, at each integer level of DLS, it is modelled - in a preferred implementation - as a mixture of *Weibull* distributions. The *Weibull* distribution was chosen due to its versatility and relative simplicity. Its probability density function is defined by two parameters, $\alpha$ and $\beta$:

$$weibull\left(x\,|\,\alpha,\beta\right) = \begin{cases} \dfrac{\alpha}{\beta}\left(\dfrac{x}{\beta}\right)^{\alpha-1} e^{-\left(\frac{x}{\beta}\right)^{\alpha}} & x \geq 0 \\ \\ 0 & x < 0 \end{cases} \qquad (1)$$

For *K* mixture components (the number of *Weibull* distributions required to model the data) and *N* retest data points $\{\mathbf{x}_n\}_{n=1}^{N}$, a latent *K*-dimensional binary vector variable $\mathbf{z}_n$ defines to which mixture component the data point $x_n$ belongs. The *k*th element $\mathbf{z}_{nk} = 1$ if $x_n$ belongs to the kth component, otherwise $\mathbf{z}_{nk} = 0$. For instance, if *K*=2 and $x_n$ belongs to the first mixture component, then vector $\mathbf{z}_n$ would be [1,0]$^\mathrm{T}$, but if $x_n$ belongs to the second it would be [0,1]$^\mathrm{T}$. With the prior probability,

$$p\left(\mathbf{z}_{nk} = 1\right) = \pi_k \qquad\qquad (2)$$

the complete likelihood of observed and latent variables becomes:

$$p\left(\mathbf{x}, \mathbf{Z} \mid \pi, \alpha, \beta\right) = \prod_n \prod_k \left(\pi_k \, weibull\left(\mathbf{x}_n \mid \alpha_k, \beta_k\right)\right)^{\mathbf{z}_{nk}} \qquad\qquad (3)$$

where $\pi$ is a vector of $\pi_k$ for $k$=1 to $K$. Note that $\pi$ is the prior probability that $x_n$ belongs to the kth mixture component and $\sum_k \pi_k = 1$. x is a vector containing $\{\mathbf{x}_n\}_{n=1}^N$, $\mathbf{Z}$ is a $K$-by-$N$ matrix containing $\mathbf{z}_n$ column vectors, $\alpha$ and $\beta$ are vectors containing parameters for the K $Weibull$ distributions defined in (1), with $\alpha_k$ and $\beta_k$ the kth components for $\alpha$ and $\beta$ respectively. Marginalising (3) over $\mathbf{Z}$ gives the likelihood of $Weibull$ mixture distribution:

$$p\left(\mathbf{x} \mid \pi, \alpha, \beta\right) = \sum_{\mathbf{Z}} p\left(\mathbf{x}, \mathbf{Z} \mid \pi, \alpha, \beta\right) = \prod_n \sum_k \pi_k \, weibull\left(\mathbf{x}_n \mid \alpha_k, \beta_k\right) \qquad\qquad (4)$$

The maximisation of (4) does not give a closed solution for parameters $\pi$, $\alpha$ and $\beta$. Therefore, an expectation-maximisation (EM) algorithm (described below in detail) was derived to iteratively optimise (4).

Further, since the log $Weibull$ distribution for the minimum DLS in visual field testing, 0dB, is undefined, a DLS $v$ was transformed to $s(v)$ such that:

$$s\left(v\right) = \begin{cases} v & v \geq 1 \\ \exp\left(v - 1\right) & v < 1 \end{cases} \qquad\qquad (5)$$

Note that $s(v)$ is $v$ itself except when $v < 1$ and the lower bound for $s(v)$ is 0dB. This transformation guarantees that the transformed DLS $s(v)$ is continuous and has a first derivative, which is an important property for the optimisation of the regression model described in the next section.

For notational simplicity, the derived retest distribution (4) for DLS $y$ is hereafter denoted as $R_y(v)$.

The spatial correlation module 33 is configured to determine spatial correlations in the historical data 43. These spatial correlations are combined with the abovedescribed $Weibull$ retest distributions outlined above in a manner that is later described.

The spatial correlation module 33 operates on the basis that given $Q$ measurements (each with $M$ test locations) in a time series at time $\{t_i\}_{i=1}^Q$, the measurement at time $t_i$ is represented as a $M$-dimensional column vector $\mathbf{y}_i$ containing elements $\{\mathbf{y}_{ij}\}_{j=1}^M$, where $\mathbf{y}_{ij}$ represents a measurement at time $i$ and location $j$. Here, $M$=52 representing a total of 52 visual field measurements in a 24-2 test grid (Fig. 4(c)). $\mathbf{Y}$ is a matrix consisting of $\{\mathbf{y}_i\}_{i=1}^Q$ in columns and represents a matrix of all measurements across all test locations and time points. To formulate the regression model in a compact notation, a column vector $\mathbf{t}_i = [t_i, 1]^T$ was used and $\mathbf{t}$ represents all $\{\mathbf{t}_i\}_{i=1}^Q$ (i.e. all time points).

The regression model is defined by weight vectors $\{\mathbf{w}_j\}_{j=0}^M$. Each $\mathbf{w}_j$ contains a slope and intercept for the $j$th location regressed over time in the case of a linear model. $\mathbf{W}$ is a matrix consisting of $\{\mathbf{w}_j\}_{j=0}^M$ column vectors (i.e. a matrix containing the slope and intercept in the fitted regression model for every location). The likelihood for measurement time series $\mathbf{Y}$ becomes:

$$p\left(\mathbf{Y} \mid \mathbf{W}, \mathbf{t}\right) = \prod_{i=1}^{Q} p\left(\mathbf{y}_i \mid \mathbf{W}, \mathbf{t}_i\right) = \prod_{i=1}^{Q} \prod_{j=1}^{M} R_{\mathbf{y}_{ij}}\left(s\left(\mathbf{w}_j^{\mathrm{T}} \mathbf{t}_i\right)\right) \qquad (6)$$

where $R_{yij}(\cdot)$ and $s(.)$ were defined in (4) and (5) respectively.

To incorporate the spatial correlation among different locations in the measurement, the expected change module 35 implements prior distributions of slope and intercepts defined to be multivariate normal distributions:

$$p\left(\mathbf{w}_a\right) = N\left(\mathbf{w}_a \mid \mathbf{m}_a, a\mathbf{\Sigma}\right) \text{ and } p\left(\mathbf{w}_b\right) = N\left(\mathbf{w}_b \mid \mathbf{m}_b, b\mathbf{\Sigma}\right) \qquad (7)$$

where $\mathbf{w}_a$ and $\mathbf{w}_b$ are the first and second rows of $\mathbf{W}$ corresponding to the slopes and intercepts of the regression lines, respectively. $\mathbf{m}_a$ and $\mathbf{m}_b$ are the means of respective normal distributions and $\Sigma$ is the covariance matrix scaled by $a$ and $b$.

Referring again to the spatial correlation module 33, the unscaled covariance matrix $\Sigma$ encodes the spatial correlation among test locations in the measurement. The element $\Sigma_{pq}$ on the pth row and $q$th column represents the strength of correlation between points $p$ and $q$. For visual field DLS measurements, $\Sigma_{pq}$ was defined as:

$$\mathbf{\Sigma}_{pq} = \begin{cases} \exp\left(-\dfrac{1}{2}\left(\dfrac{dist_{pq}^2}{2\delta_d^2} + \dfrac{\angle_{pq}^2}{2\delta_\angle^2}\right)\right) & \text{if p and q both lie in the same} \\ & \text{hemifield (upper or lower)} \\ 0 & \text{otherwise} \end{cases} \qquad (8)$$

where $dist_{pq}$ is the Euclidian distance between the points $p$ and $q$ in the visual field, and $\angle_{pq}$ is the difference between the angles that the optic nerve fibres crossing points $p$ and $q$ enter the optic nerve head $\delta_d$ and $\delta_\angle$ are scale parameters chosen to be $\delta_d = 6°$ and $\delta_\angle = 14°$, according to the distance between two neighbouring points in the visual field and the 95% confidence interval of population variability in the nerve fibre entrance angle into the optic nerve head, respectively. Note that $\Sigma_{pq} = 0$ if the two points lie on different hemifields (upper or lower; Fig. 4(c)) of the visual field due to the physiological distribution of optic nerve fibres. The unscaled covariance $\Sigma$ between each location in the visual field and all other points is illustrated in Fig. 6.

In this particular example the values for the scale parameters $a$ and $b$ in the expected change module 35 were chosen to produce non-informative priors for $\mathbf{w}_a$ and $\mathbf{w}_b$. To be exact, the slope prior was set as $\mathbf{m}_a = 0$ (dB/year) with $a = 10^2$ corresponding to a slope standard deviation of 10dB/year. The intercept prior is set such that $\mathbf{m}_b$ equals the median of the measurements at each location in the series and $b = 10^2$ corresponding to an intercept standard deviation of 10dB.

The target function construction module 37 combines the output of the modules described above. According to (6) and (7), the log of posterior probability of $\mathbf{W}$ **can** be derived as:

$$\ln p\left(\mathbf{W} \mid \mathbf{Y}, \mathbf{t}\right) = \sum_{i=1}^{N} \sum_{j=1}^{M} R_{\mathbf{y}_{ij}}\left(s\left(\mathbf{w}_j^{\mathrm{T}} \mathbf{t}_i\right)\right) - \frac{1}{2}\left(\mathbf{w}_a - \mathbf{m}_a\right)^{\mathrm{T}} \mathbf{\Lambda}_a \left(\mathbf{w}_a - \mathbf{m}_a\right)^{\mathrm{T}}$$
$$-\frac{1}{2}\left(\mathbf{w}_b - \mathbf{m}_b\right)^{\mathrm{T}} \mathbf{\Lambda}_b \left(\mathbf{w}_b - \mathbf{m}_b\right)^{\mathrm{T}} + const \qquad (9)$$

where $\mathbf{\Lambda}_a^{-1} = a\mathbf{\Sigma}$, $\mathbf{\Lambda}_b^{-1} = b\mathbf{\Sigma}$. The terms independent of $\mathbf{W}$ are grouped into the constant term, *const.*

The optimisation module 39 is configured to determine a fit for the analytical function to an input time series dataset 45 for a particular patient, and output a change model for the analysed time series dataset.

The posterior probability (9) cannot be recognised as a known distribution because (7) is not the conjugate prior of the mixture of *Weibull* distributions. Although the log posterior (9) can still be maximised with regard to $\mathbf{W}$, it is difficult to estimate the exact variance of $\mathbf{W}$ without knowing the underlying distribution. Therefore, a *Laplace* approximation was used to approximate $p(\mathbf{W} \mid \mathbf{Y}, \mathbf{t})$ as a normal distribution centred at the mode of $\mathbf{W}$, as described below in more detail. The estimates of the slope and intercept in the *Laplace* method exactly match the local maximum of log posterior probability (9). However, the variance of these slopes and intercepts are approximate estimates.

The technique described above differs from conventional linear regression in two ways. As opposed to the normally distributed noise assumed in ordinary linear regression, the technique disclosed respects the actual variability associated with measurements through making use of the quantified retest distribution. Moreover, the spatial correlation among test locations in the visual field is taken into account by the prior probability defined in (7). It is also the case that for the purpose of evaluating the effects of spatial correlation, its contribution can be 'switched off by setting the off-diagonal elements of $\Sigma$ in (7) to be 0.

The reporting module 41 is configured to produce various items of useful information about change in the time series depending on the particular application. Example of such information includes: localisation of change over time (e.g. probability and rate of change at one or more or each location in measurement time series), summarisation of change over time (e.g. probability that change is happening somewhere in the measurement time series). The inferred change statistics (e.g. rate of change and the corresponding confidence intervals) can also be used to predict measurement in the future, e.g. by projecting the measurement to the future time according to the current estimate of rate of change.

The particular implementation described herein estimates the slope and intercept with their variance approximated by the *Laplace* method (further details of which are provided below). In alternative implementations variational approximation or expectation propagation may be employed instead of the aforementioned *Laplace* method.

The distribution of the slope is of particular clinical importance because it represents the rate and certainty of change. The 'change' applies equally to deterioration (negative change) and improvement (positive change) in measurements. In the case of a progressive condition, such as glaucoma, the slope distribution at each location can be summarised as the 'probability of no-deterioration', which is quantified as the cumulative distribution of slope $\geq$0dB/year. The 'probability of no-deterioration' value will be referred to as *Pnd* hereafter. The *Pnd* value ranges between 0 and 1 where a lower value indicates a higher probability of deterioration.

In order to summarise the possibility of deterioration across all *M* test locations in the visual field series, a global index, the deterioration index $I^-$, is defined as:

$$I^- = -\sum_{j=1}^{M} \ln Pnd_j \qquad (10)$$

where $Pnd_j$ is the *Pnd* value at the jth location in the measurement. $I^-$ is the negative logarithm of the product of all *Pnd* values, thus, non-negative and larger value implies greater certainty about deterioration in the measurement series. Similarly, to evaluate the improvement in a measurement series, such as in the case of gene therapy for retinal disease, the improvement index $I^+$ can be derived as $I^+ = -\sum_{j=1}^{M} \ln\left(1 - Pnd_j\right)$. However, because this illustrative application of the teachings of the invention concerns the identification of deterioration in retinal function in glaucoma, the index will henceforth only refer to $I^-$.

As mentioned above, maximising the likelihood (4) does not provide a closed solution for the estimation of $\pi$, $\alpha$ and $\beta$, so an expectation-maximisation (EM) algorithm was used to form an iterative optimisation. The EM algorithm is a widely used parameter estimation framework that starts with initial values of parameters and iterates between the expectation (E-) and maximisation (M-) steps until convergence. Convergence was indicated by an absolute difference of log marginal distribution (4) of less than $10^{-6}$ between two consecutive iterations.

The EM algorithm was initialised through the selection of starting values for parameters $\pi$, $\alpha$ and $\beta$. $\pi_k = \frac{1}{K}$ and $\alpha$ and $\beta$ were set as random numbers in the range between 5 and 20. In the E-step, the expectation of $\mathbf{z}_{nk}$ under the distribution $p(\mathbf{Z} \mid \mathbf{X}, \pi^{old}, \alpha^{old}, \beta^{old})$ is evaluated as:

$$\gamma_{nk} = \mathrm{E}_{p\left(\mathbf{Z}|\mathbf{X},\pi^{old},\alpha^{old},\beta^{old}\right)}\left(\mathbf{z}_{nk}\right) = \frac{\pi_k\, weibull\left(\mathbf{x}_n \mid \alpha_k^{old},\beta_k^{old}\right)}{\sum_{k'} \pi_{k'}\, weibull\left(\mathbf{x}_n \mid \alpha_{k'}^{old},\beta_{k'}^{old}\right)} \qquad (11)$$

where $\alpha^{old}$ and $\beta^{old}$ are initial *Weibull* distribution parameters if the E-step is in the first iteration, or otherwise are estimated parameters from the previous M-step.

The M-step maximises the expectation of complete log likelihood (3) ln $p(\mathbf{X}, \mathbf{Z} \mid \pi, \alpha, \beta)$ under the distribution $p(\mathbf{Z} \mid \mathbf{X}, \pi^{old}, \alpha^{old}, \beta^{old})$ with respect to $\pi$, $\alpha$ and $\beta$ :

$$E_{p\left(\mathbf{Z}|\mathbf{X},\boldsymbol{\pi}^{old},\boldsymbol{a}^{old},\boldsymbol{\beta}^{old}\right)}\left(\ln p\left(\mathbf{x},\mathbf{Z}\mid\boldsymbol{\pi},\boldsymbol{a},\boldsymbol{\beta}\right)\right)=\sum_{n}\sum_{k}\gamma_{nk}\left(\ln\boldsymbol{\pi}_{k}+\ln weibull\left(\mathbf{x}_{n}\mid\boldsymbol{a}_{k},\boldsymbol{\beta}_{k}\right)\right) \qquad (12)$$

where $\gamma_{nk}$ was evaluated in by the E-step. Maximizing with respect to $\pi_k$ under the constraint $\sum_{k}\boldsymbol{\pi}_{k}=1$ using a Lagrange multiplier gives:

$$\boldsymbol{\pi}_{k}=\sum_{n}\gamma_{nk} \qquad (13)$$

Because there is no closed solution for $\alpha$ and $\beta$, the maximisation of with respect to $\alpha$ and $\beta$ is carried out using the Quasi-Newton algorithm. This avoids the exact computation of the Hessian matrix of parameters as required by the Newton's method. The Quasi-Newton algorithm makes use of the gradient of with respect to $\alpha$ and $\beta$, which can be derived from (1) and . The Quasi-Newton algorithm was set to stop when the absolute change of in two consecutive iterations was lower than $10^{-6}$.

Referring again to the aforementioned Laplace method for approximating for posterior distribution $p(\mathbf{W}|\mathbf{Y},t)$, in a first step a Taylor expansion of (9) is taken around the mode $\mathbf{W}_{max}$ of (9). For the purpose of optimisation, $\mathbf{W}$ and $\mathbf{W}_{max}$ are treated as vectors.

$$\ln p\left(\mathbf{W}\mid\mathbf{Y},\mathbf{t}\right)\approx\ln p\left(\mathbf{W}^{max}\mid\mathbf{Y},\mathbf{t}\right)+\left(\mathbf{W}-\mathbf{W}^{max}\right)^{\mathbf{T}}\nabla\ln p\left(\mathbf{W}^{max}\mid\mathbf{Y},\mathbf{t}\right)$$
$$-\frac{1}{2}\left(\mathbf{W}-\mathbf{W}^{max}\right)^{\mathbf{T}}\mathbf{H}\left(\mathbf{W}-\mathbf{W}^{max}\right) \qquad (14)$$

where $\mathbf{H}=-\nabla\nabla\log p(\mathbf{W}\mid\mathbf{Y},\mathbf{t})$ is the negative Hessian matrix of (9). Because $\mathbf{W}_{max}$ is a saddle point of $\log p(\mathbf{W}\mid\mathbf{Y},t)$, $\nabla\log p(\mathbf{W}^{max}\mid\mathbf{Y},\mathbf{t})=0$ and the second term in becomes 0. In this case, $\ln p(\mathbf{W}\mid\mathbf{Y},\mathbf{t})$ is approximated as a quadratic function of $\mathbf{W}$, indicating that $p(\mathbf{W}\mid\mathbf{Y},\mathbf{t})$ approximately follows a normal distribution:

$$p\left(\mathbf{W}\mid\mathbf{Y},\mathbf{T}\right)\approx N\left(\mathbf{W}\mid\mathbf{W}^{max},\mathbf{H}^{-1}\right) \qquad (15)$$

There are two steps in the *Laplace* approximation. First, $\mathbf{W}_{max}$ of (9) is estimated by a scaled conjugate gradient algorithm, with initial parameters of $\mathbf{W}$ set as $\mathbf{m}_a$ and $\mathbf{m}_b$ in (7). The scaled conjugate gradient algorithm uses the gradient of (9) derived by differentiation using the chain rule and iteratively searches for $\mathbf{W}_{max}$, which maximises (9). The algorithm stops when the absolute change of the objective function (9) in two consecutive iterations is less than $10^{-6}$. Secondly, the Hessian matrix of (9) at $\mathbf{W}_{max}$ and the approximated covariance matrix $\mathbf{H}^{-1}$ are calculated.

In a preferred implementation, to extrapolate beyond the maximum series length of the test-retest dataset, the threshold was modelled as a sigmoidal function of the series length (*length*) with its magnitude modulated by an exponential function of false positive rate (*FP*) between 2% and 20%:

$$threshold=\frac{(m\cdot e^{p\cdot FP}+n\cdot e^{q\cdot FP})}{(1+e^{-a\cdot length-b})} \qquad (16)$$

The regression model was optimised by the *Trust-Region* algorithm and provided a very good fit ($R^2$=0.99). The fitted $\Gamma$ thresholds from with various false positive rates and series lengths are presented in Fig. 14, which can be used as a look-up table to calculate $\Gamma$ threshold given false positive rate and series length.

The techniques described above have been extensively evaluated. In that evaluation, all visual fields were measured via SAP with the Humphrey Visual Field Analyzer (HFA, Carl Zeiss Meditec, CA, USA) using the 24-2 test pattern (Fig. 4(c)) and the SITA (Swedish Interactive Thresholding Algorithm) Standard testing algorithm. The test measures retinal DLS at about 50 test locations, where each test location is evenly separated by an angular distance of 6° across the visual field (Fig. 4(c)).

Two datasets collected at different centres were used. The first dataset was from a study examining the 'test-retest' variability of SAP conducted at Dalhousie University, Halifax Canada in a cohort of glaucoma patients. Changes in retinal function are slow in glaucoma. By taking a series of measurements in a short period of time, it is possible to estimate measurement test variability, under the assumption that no measurable deterioration can occur over the observation period. One eye of 30 patients was tested 12 times over a short period (maximum 8 weeks). For our purposes, each of these visual field series, and the same series with arbitrary reordering, represents a "stable" series with no underlying deterioration.

The second dataset was sampled from 402,357 visual fields of 75,857 patients from clinical practice at Moorfields Eye Hospital in London. The first visual field of each series was discarded to limit the impact of "learning effects" and if multiple visual fields were taken on the same day, the last measurement was chosen. Only series that were obtained over 6 years and contained at least 7 visual fields were included in the study. Note that the length of series is purely for evaluation purposes and is not necessitated by the proposed model. The resulting dataset consisted of 47,483 visual field tests from 6,011 series from 6,011 eyes, representing about 2.5 million individual DLS measurements. The median (interquartile range [IQR]) time of follow up was 9.3 (7.9, 10.4) years and the median (IQR) number of visual fields in each time series was 9 (8, 11). The median (IQR) interval between visual field tests was 1.0 (0.6, 1.4) years.

To evaluate the utility of the techniques described herein in detecting retinal function change, it is necessary to compare it to other change detection methods currently used in clinical decision making. Point-wise linear regression, the most widely used method, fits an ordinary linear regression model to a time series of measurement for each location in the visual field and assesses the significance and slope of the fit. Summary measures, such as the mean deviation from the average DLS of healthy eyes, are also often utilised, but since glaucoma tends not to affect all locations to the same extent, global indices often have inadequate statistical sensitivity to detect worsening when compared with methods assessing deterioration at individual locations. Thus, the present technique was compared with three other methods: ordinary linear regression of mean deviation, point-wise linear regression and an implementation of the present technique without spatial enhancement.

A false positive is a type I error where change is falsely detected in a series with no true deterioration. The false positive rate can be reliably estimated in a series of repeated measurements acquired in a period of time too short for measureable deterioration; additional reordering of repeated measurements also produces pseudo-series with no true deterioration.

The series of 12 visual fields from 30 eyes in the test-retest dataset were randomly reordered 300 times, so 90,000 pseudo-series of length between 3 and 12 were generated. It was assumed that one visual field measurement per year was taken in these pseudo series (the median test frequency in the Moorfields dataset). The false positive rate was then estimated as the proportion of series identified as deteriorating. In a clinical situation, false positives may lead to overtreatment and unnecessary cost, so methods with high false positive rates are generally considered as not clinically useful. Moreover, in an investigation of a new therapy for preserving visual function, high false positive change would lead to erroneous findings.

When evaluating the sensitivity and time to detect change, different methods should be compared at equivalent false positive rates. The false positive rate is dependent on the chosen change criterion, for instance, based on the threshold of the index $I$ and the length of the series. In the following evaluation of various methods, the criteria given were formulated such that the false positive rate for each method was identical in the test-retest dataset for different series lengths. These criteria were then applied on measurement series from clinical practice to evaluate the performance of each method.

For ordinary linear regression of mean deviation, deterioration criteria were a negative slope and p-value lower than a set threshold. For point-wise linear regression, deterioration criteria for each test location were a negative slope and a p-value<1% and the visual field was worsening when at least n=1, 2, 3 and 4 contiguous points were deteriorating. For the present technique and a modification of that technique that is without spatial enhancement, the criterion for deterioration was a $I$ value higher than a given threshold. The thresholds were chosen to equate false positive rates.

Early detection of deterioration prompts clinical intervention which aims to prevent further vision loss, so is particularly important for the management of chronic conditions. Moreover, being able to detect change in retinal function reliably in short period of time ensures that clinical trials of new therapies can be done rapidly. Therefore, the time to detect deterioration was compared between methods using the dataset from clinical practice at Moorfields Eye Hospital.

In each visual field series, a subseries containing the first three visual fields was considered as the minimum series length required to detect change. The length of the subseries was then increased by incrementally adding visual fields to the subseries in chronological order. The shortest series that was flagged as deteriorating was then recorded for each method. If no deterioration was detected in any subseries of a visual field series by a method, the time was recoded as the total time span of the series.

Because any subseries flagged as deteriorating may be a false positive, it was important to control the false positive rate when comparing the performances of deterioration detection methods. Therefore, the time efficiency of the methods was compared at various fixed false positive rates.

Statistical sensitivity is the measure of a method's ability to identify true change. Ideally, the sensitivity should be evaluated as the proportion of detected change in the visual field series with true underlying deterioration. However, due to the lack of a 'gold-standard' and 'ground-truth' classification for glaucomatous deterioration, the underlying worsening status of each visual field series was unknown. Therefore, all visual field series from the Moorfields dataset, regardless of their deterioration status, were used to estimate the hit rate, which was the proportion of series flagged as deteriorating. This is justified if the false positive rate is controlled to be equivalent for all methods. Particularly, given an unknown proportion $p\%$ of truly worsening series in the dataset, the hit rate is equal to ($p\%\times$sensitivity)+[(1-$p\%$)$\times$false positive rate]. At equal false positive rates, a higher hit rate implies better sensitivity of a method.

Therefore, hit rates of all methods were compared at various corresponding false positive rates and series lengths as a surrogate comparison for sensitivity. Furthermore, DLS deteriorates as a result of ageing, and cannot increase in response to standard medical treatments for glaucoma. Thus, all series in the dataset should be worsening at a rate at least equal to age-related decline. When positive rates are observed, in the case of glaucoma, this is usually due to "learning effects" or the inherent variability of the measurement.

The technique described in detail herein was implemented in MATLAB R2011b (MathWorks Inc., Natick, MA). Analysis of a series with 10 visual fields took approximately 1.5 seconds on a 2.50GHz Intel i7 processor.

Two mixture components were used to model retest distribution for sensitivities between 0dB and 35dB. The histograms and the derived probability density functions of the *Weibull* mixture at different DLSs are shown in Fig. 7. Despite the non-stationary variability, each distribution can be sufficiently described by a combination of two *Weibull* distributions.

Compared with the normal distribution of error assumed by ordinary linear regression, the quantified retest distribution used in *ANSWER* takes into account the non-stationary variability in visual field DLS measurements. The examples in Fig. 8 demonstrate the difference between the present technique and ordinary linear regression in series of DLSs at a single visual field location. Because only a single visual field location was considered for illustrative purposes, there is no spatial enhancement in these examples.

In Fig. 8(a), the last measurement in the series changes suddenly due to measurement variability, leading to a steep slope in the ordinary linear regression. By comparison, a modification of the present technique (without spatial enhancement) is less affected by the last measurement since it accounts for the large variability associated with measurements at this level of DLS, and so results in a shallower slope. This property of this technique makes it robust to the non-stationary variability of DLS measurements and, therefore, a more reliable estimator of deterioration rate.

Despite the robustness of this technique, it does not compromise sensitivity to detect deterioration. In fact, by taking into account the non-stationary variability of DLS measurements, the method is able to detect significant deterioration in short time series where conventional methods cannot reach statistical significance. In Fig. 8(b), ordinary linear regression did not indicate significant deterioration ($p$-value>5%), while this technique managed to ascertain with high certainty that deterioration was occurring ($Pnd$<0.1%). This property allows this technique to provide better time efficiency in detecting deterioration.

Fig. 9 shows the average time to first detect deterioration in the visual field series with each method at false positive rates between 0 and 15%. Because the criteria for point-wise linear regression (based on the number of contiguous points with deterioration in the visual field) are integer values (not continuous), the time efficiency of point-wise linear regression could not be estimated with a continuous false positive rate. Moreover, the false positive rate with the single-point criterion of point-wise linear regression was higher than 15%, so this is not shown in Fig. 9.

For each method, the time to detection change was compared at the 5% false positive rate, or at the closest rate to 5% for point-wise linear regression (2 contiguous points, false positive rate of 6%). At this false positive rate, this technique detected deterioration faster than point-wise linear regression ($p$<0.1% paired t-test) and linear regression of mean deviation ($p$<0.1% paired t-test). Furthermore, with spatial enhancement, this technique was able to detect deterioration significantly faster than without ($p$<0.1% paired t-test). On average, the technique with spatial enhancement detected deterioration 2.12 (95% confidence interval [2.05, 2.19]) years ahead of point-wise linear regression, 2.19 (95% confidence interval [2.11, 2.26]) years before linear regression of mean deviation, and 0.40 (95% confidence interval [0.35, 0.44]) years before the technique without spatial enhancement.

The hit rates of the four methods were estimated with various series lengths and at false positive rates between 0 and 15%. Fig. 10 demonstrates the hit rate with series lengths of 3, 5, 7 and 9. The methods were also compared at the 5% false positive rate, or at the closest rate to 5% for point-wise linear regression (two contiguous points criterion). The ratios of hit rates between pairs of methods are shown in where a ratio >1 indicates a better hit rate. For instance, with series of 7 visual fields, the ratio of the present technique against linear regression of mean deviation was 2.0, indicating that the hit rate of the present technique is twice that of the latter method.

The hit rates of the present technique were higher than linear regression of mean deviation and point-wise linear regression of DLS at all series lengths. There was particular improvement in short series, indicating that the present technique can detect a higher proportion of deterioration with short visual field series than the other two conventional methods. This explains the better efficiency of the present technique for detecting deterioration more quickly. Including spatial enhancement also increased the hit rate, especially with short series.

Fig. 11 demonstrates the proficiency of the present technique at detecting change in a time series with only 3 visual fields. This is for demonstrative purpose and in clinical situation decision should not be solely based on series as short as three measurements. The last three visual fields were simply shown as further evidence of the deterioration status. The linear regression of mean deviation and point-wise linear regression did not reach statistical significance to alert worsening. The present technique, on the other hand, detected early and rapid deterioration mainly in the superior and the nasal-superior regions of the visual field. This early defect aggressively developed into local blindness in about three years. For the purpose of visualisation, *Pnd* values <5% are indicated in red, suggesting a high likelihood of deterioration. The index $I$ of 181 for the present technique is above the threshold corresponding to the 5% false positive rate (82 for this series length).

Visual field measurements may improve with a patient's experience. This 'learning effect' may result in an apparent overall improvement in the visual field, which may mask local deterioration. As an example, Fig. 12 shows a visual field series with increasing mean deviation as the patient gains experience with the test. Point-wise linear regression of DLS does not confirm localized deterioration at contiguous points. However, despite an overall apparent improvement in the visual field, the present technique detects rapid deterioration in the local nasal region and deterioration in the nasal-superior and inferior regions; the index, $I$, for the present technique is equal to 128 and is higher than the threshold at the 5% false positive rate (in this case, 89).

To demonstrate the benefit of taking into account spatial correlation, especially in short visual field series, an example of the present technique with and without spatial enhancement is given in Fig. 13. The technique with spatial enhancement detects a large number of deteriorating points by aggregating information from adjacent measurements according to their spatial correlation. At the 5% false positive rate, the index $I$ of 98 for the spatially enhanced technique was above the threshold of 82, while the $I$ of 69 for the non-spatially enhanced technique was below the threshold of 97.

It can be seen from the foregoing that the present technique detected change in retinal function more rapidly than conventional statistical approaches without compromising false positive rates. At equivalent false positive rates, it also detected a greater number of eyes with change in retinal function when compared to the number detected by other widely used methods. The Weibull mixture retest distributions, in comparison to a normally distributed error assumed in ordinary regression models, allows the present technique to attain a high certainty about deterioration status (Fig. 8(b)). In addition, the spatial enhancement aggregates information for adjacent locations in the visual field to 'confirm' the spatial deterioration pattern, further improving the method especially for short time series. This spatial element of detecting change in visual fields has rarely been considered before. The present technique could not only aid clinical decision for prompt treatment intervention, but also define more efficient endpoints for clinical trials in eye-related research.

The application and usefulness of the present technique in short series is of particular clinical interest. Current widely used methods typified by ordinary linear regression for change detection are limited in short series because they can hardly reach required statistical significance. In clinical situations, where follow-up testing is infrequent, often due to limited resources, these standard analyses may delay the detection of change in retinal function. In turn this can delay required intensification of treatment. In clinical trials, failing to pick up change in time could also lengthen the trials.

When choosing thresholds for the present technique to detect deterioration in visual field series, it is critical to consider the false positive rate for the chosen threshold of $I$. In this study, the threshold was estimated from the test-retest dataset at given false positive rates and for each visual field series length.

The *Laplace* method used in the preferred arrangement provides local normal approximation at the mode of the posterior slope and intercept distribution (9), so estimations of variance of these regression parameters may not capture every feature of the distribution (skewness for example). Although the true posterior distribution (9) is unknown, the estimated slope variance from the *Laplace* approximation was nonetheless demonstrated to be an effective variable in detecting change and quantifying the certainty about change relative to other current methods.

The present technique was developed with the idea that it could be adapted for other applications with similar statistical properties which are not uncommon among other biological and medical measurements. For example, serum creatinine measurement for predicting kidney failure, heart rate measurement for assessing heart attack risk and baroreceptor sensitivity feedback in diabetes mellitus pose similar challenges in clinical decision making. There are two necessary steps in order to adapt the present technique to other types of clinical measurement. First, the non-stationary variability or retest distribution should be derived from the measurement in question; since the *Weibull* distribution is versatile and concise, it could easily be adjusted to model other retest distributions. Second, the spatial correlation (8) would need to be adapted (or removed if necessary) according to the properties of the measurement, as demonstrated in this study. The present technique was used to infer linear change because there are generally insufficient data to identify non-linear change due to the short visual field series in clinical practice; however, configuring the present technique to measure change of conditions with long series and temporal processes showing non-linear change is trivial, and can be done by changing the time vector $\mathbf{t}_i$ in (6) to nonlinear components such as radial basis functions.

In conclusion, the present technique provides a solution in a landscape of uncertainty in detecting retinal function deterioration. This could, for example, impact on how patients with glaucoma are monitored and treated and the efficiency and duration of clinical trials. The present technique was found to outperform conventional methods of detecting retinal function deterioration both in terms of statistical sensitivity, and in time taken to detect change.

In one illustrative implementation the techniques described herein may be implemented in software (for example, software running on a stand-alone computer, or software running on specialised equipment (such as a Humphrey Visual Field Analyzer)).

It will be appreciated that whilst various aspects and embodiments of the present invention have heretofore been described, the scope of the present invention is not limited to the particular arrangements set out herein.

For example, whilst the teachings of the present invention may be implemented (wholly or partly) in software, it will be apparent to persons of ordinary skill in the art that the teachings of the invention may be implemented in hardware (for example in one or more ASICs), or indeed in a combination of hardware and software.

Lastly, it should also be noted that the scope of the present invention is not limited to the particular combinations of features explicitly disclosed herein, but instead extends to encompass any combination of features as defined in the claims.

**Claims**

1. A change analysis system (29) for a time series dataset concerning retinal structure and/or function, the system comprising:

   an error profiling module (31) configured to receive a historical set of data (43) as an input, to determine errors in said historical set of data, said historical set of data comprising a plurality of visual field measurements taken at spatially distributed retinal test locations over a period of time, and to output an analytical model of said errors;
   a spatial correlation quantification module (33) configured to receive said historical set of data as an input, to determine a spatial correlation among the retinal test locations in said historical set of data, and to output an analytical model of said spatial correlations;
   an expected change module (35) configured to generate an analytical model of expected changes, said model comprising an expectation of changes in a time series dataset concerning visual field for a particular patient that is to be analysed by said change analysis system;
   a target function construction module (37) configured to receive as an input a time series data set concerning visual field measurements for a particular patient, to construct from said error model,
   and spatial correlation model, an analytical function representing a probability of change in said time series dataset;
   an optimisation module (39) configured to determine a fit for said constructed analytical function to said time series dataset, and output a change model for the analysed time series dataset; and
   a reporting module (41) configured to generate from said change model a report concerning changes in retinal function in said analysed dataset.

2. A system according to Claim 1, wherein said reporting module is operable to generate a report concerning the rate of change in said analysed dataset, and/or to generate a report concerning a probability of change in said analysed dataset, said probability of change comprising a probability of change having already occurred or a probability of a change occurring at some point in the future.

3. A system according to any preceding claim, further comprising an expected change module configured to generate an analytical model of expected changes, said model comprising an expectation of changes in a time series dataset that is to be analysed by said change analysis system; wherein said target function construction module configured to construct from said error model, said expected change model and said spatial correlation model, an analytical function representing a probability of change in said time series dataset;

4. A system according to any preceding claim, wherein said expected change module is configured to generate said model of expected changes from changes in another set of data that is related to the dataset to be analysed such as data pertaining to a different type of test to a test or tests from which said dataset to be analysed resulted.

5. A system according to Claim 4, wherein one of said dataset to be analysed and said another set of data comprises data from one or more functional tests that measure the function of an object, and the other of said dataset to be analysed and said another set of data comprises data from one or more structural tests that measure the structure of an object.

6. A system according to any preceding claim, wherein said error profiling module is operable to model heteroscedastic and/or non-normal error.

7. A system according to Claim 6, wherein said error profiling module is operable to fit one or a mixture of Weibull distributions to said historical data set.

8. A system according to any preceding claim, wherein said errors comprise variability in measurements of a measured object over time and/or noise, said noise comprising differences between true measurements and recorded measurements.

9. A system according to any preceding claim, wherein said spatial correlation quantification module is configurable for a particular condition, such as glaucoma or macular degeneration.

10. A system according to any preceding claim, wherein said time series dataset that is to be analysed comprises a set of data output from a testing device.

11. A system according to any preceding claim, wherein said target function construction module is operable to implement a Bayesian framework, and/or said expected change module outputs prior probability distributions in a Bayesian framework.

12. A testing device, such as a visual field perimeter or a retinal structure imaging device, comprising a system according to any preceding claim.

13. A change analysis method for a time series dataset concerning retinal structure and/or function, the method comprising:

by means of an error profiling module (31), receiving historical data as an input, determining errors in said historical set of data; said historical set of data comprising a plurality of visual field measurements taken at spatially distributed retinal test locations over a period of time, and outputting an analytical model of said errors; by means of a spatial correlation quantification module (33), receiving as an input said historical set of data, determining a spatial correlation among the test locations in said historical set of data and outputting an analytical model of said spatial correlations; by means of a target function construction module (37), receiving as an input a time series data set concerning visual field measurements for a particular patient, constructing from said error model and said spatial correlation model, an analytical function representing a probability of change in said time series dataset; by means of an optimisation module (39), determining a fit for said constructed analytical function to said time series dataset, and outputting a change model for the analysed time series dataset; and by means of a reporting module (41), generating from said change model a report concerning changes in retinal function in said analysed dataset.

14. A method according to Claim 13, wherein one or more of the method steps are accomplished at a first location remote from a second location where said time series dataset has been created.

15. A computer program comprising instructions to cause the system of claim 1 to implement the steps of the method of Claims 13 or 14.

**Patentansprüche**

1. Änderungsanalysesystem (29) für einen Zeitreihendatensatz betreffend Netzhautstruktur und/oder -funktion, wobei das System Folgendes umfasst:

ein Fehlerprofilierungsmodul (31), das konfiguriert ist zum Empfangen eines historischen Satzes von Daten (43) als Eingabe, zum Bestimmen von Fehlern in dem historischen Satz von Daten, wobei der historische Satz von Daten eine Vielzahl von Gesichtsfeldmessungen umfasst, die an räumlich verteilten Netzhautteststellen über einen Zeitraum vorgenommen worden ist, und zum Ausgeben eines analytischen Modells der Fehler; ein Modul (33) zur Quantifizierung von räumlichen Korrelationen, das konfiguriert ist zum Empfangen des historischen Satzes von Daten als Eingabe, zum Bestimmen einer räumlichen Korrelation zwischen den Netzhautteststellen in dem historischen Satz von Daten und zum Ausgeben eines analytischen Modells der räumlichen Korrelationen;

ein Modul (35) für eine erwartete Änderung, das konfiguriert ist zum Generieren eines analytischen Modells von erwarteten Änderungen, wobei das Modell eine Erwartung von Änderungen in einem Zeitreihendatensatz betreffend ein Gesichtsfeld für einen konkreten Patienten umfasst, der durch das Änderungsanalysesystem zu analysieren ist;

ein Zielfunktionsaufstellungsmodul (37), das konfiguriert ist zum Empfangen eines Zeitreihendatensatzes betreffend Gesichtsfeldmessungen für einen konkreten Patienten als Eingabe, zum Aufstellen einer analytischen Funktion, die eine Wahrscheinlichkeit einer Änderung in dem Zeitreihendatensatz darstellt, aus dem Fehlermodell und Modell räumlicher Korrelationen;

ein Optimierungsmodul (39), das konfiguriert ist zum Bestimmen einer Anpassung für die aufgestellte analytische Funktion an den Zeitreihendatensatz und Ausgeben eines Änderungsmodells für den analysierten Zeitreihendatensatz; und

ein Berichtsmodul (41), das konfiguriert ist zum Generieren eines Berichts betreffend Änderungen der Netzhautfunktion in dem analysierten Datensatz aus dem Änderungsmodell.

2. System nach Anspruch 1, wobei das Berichtsmodul betreibbar ist zum Generieren eines Berichts betreffend die Rate einer Änderung in dem analysierten Datensatz und/oder zum Generieren eines Berichts betreffend eine Wahrscheinlichkeit einer Änderung in dem analysierten Datensatz, wobei die Wahrscheinlichkeit einer Änderung eine Wahrscheinlichkeit, dass eine Änderung bereits aufgetreten ist, oder eine Wahrscheinlichkeit, dass eine Änderung irgendwann in der Zukunft auftritt, umfasst.

3. System nach einem vorhergehenden Anspruch, ferner umfassend ein Modul für eine erwartete Änderung, das konfiguriert ist zum Generieren eines analytischen Modells von erwarteten Änderungen, wobei das Modell eine Erwartung von Änderungen in einem Zeitreihendatensatz umfasst, der durch das Änderungsanalysesystem zu analysieren ist; wobei das Zielfunktionsaufstellungsmodul konfiguriert ist zum Aufstellen einer analytischen Funktion, die eine Wahrscheinlichkeit einer Änderung in dem Zeitreihendatensatz darstellt, aus dem Fehlermodell, dem Modell von erwarteten Änderungen und dem Modell räumlicher Korrelationen.

4. System nach einem vorhergehenden Anspruch, wobei das Modul für eine erwartete Änderung so konfiguriert ist, dass es des Modells der erwarteten Änderungen aus Änderungen in einem anderen Satz von Daten generiert, der sich auf den zu analysierenden Datensatz bezieht, wie etwa Daten, zu einem anderen Testtyp oder Test, aus denen der Datensatz zu analysiert werden soll resultierte.

5. System nach Anspruch 4, wobei einer von dem zu analysierenden Datensatz und dem anderen Satz von Daten Daten aus einem oder mehreren Funktionstests umfasst, die die Funktion eines Objekts messen, und der andere von dem zu analysierenden Datensatz und dem anderen Satz von Daten umfassen Daten aus einem oder mehreren Strukturtests, die die Struktur eines Objekts messen.

6. System nach einem vorhergehenden Anspruch, wobei das Fehlerprofilierungsmodul betreibbar ist zum Modellieren eines heteroskedastischen und/oder nicht normalen Fehlers.

7. System nach Anspruch 6, wobei das Fehlerprofilierungsmodul betreibbar ist zum Anpassen einer oder einer Mischung von Weibull-Verteilungen an den historischen Datensatz.

8. System nach einem vorhergehenden Anspruch, wobei die Fehler Variabilität in Messungen eines gemessenen Objekts im Lauf der Zeit und/oder Rauschen umfassen, wobei das Rauschen Unterschiede zwischen wahren Messungen und aufgezeichneten Messungen umfasst.

9. System nach einem vorhergehenden Anspruch, wobei das Modul zur Quantifizierung von räumlichen Korrelationen für eine konkrete Krankheit, wie etwa Glaukom oder Makuladegeneration, konfigurierbar ist.

10. System nach einem vorhergehenden Anspruch, wobei der zu analysierende Zeitreihendatensatz einen aus einer Testvorrichtung ausgegebenen Satz von Daten umfasst.

11. System nach einem vorhergehenden Anspruch, wobei das Zielfunktionsaufstellungsmodul betreibbar ist zum Implementieren eines Bayesschen Rahmenwerks und/oder das Modul für eine erwartete Änderung früherer Wahrscheinlichkeitsverteilungen in einem Bayesschen Rahmenwerk ausgibt.

**12.** Testvorrichtung, wie etwa Gesichtsfeldperimeter oder Bildgebungsvorrichtung für Netzhautstruktur, umfassend ein System nach einem vorhergehenden Anspruch.

**13.** Änderungsanalyseverfahren für einen Zeitreihendatensatz betreffend Netzhautstruktur und/oder -funktion, wobei das Verfahren Folgendes umfasst:

mittels eines Fehlerprofilierungsmoduls (31) Empfangen von historischen Daten als Eingabe, Bestimmen von Fehlern in dem historischen Satz von Daten; wobei der historische Satz von Daten eine Vielzahl von Gesichtsfeldmessungen umfasst, die an räumlich verteilten Netzhautteststellen über einen Zeitraum vorgenommen worden ist, und Ausgeben eines analytischen Modells der Fehler;

mittels eines Moduls (33) zur Quantifizierung von räumlichen Korrelationen Empfangen des historischen Satzes von Daten als Eingabe, Bestimmen einer räumlichen Korrelation zwischen den Teststellen in dem historischen Satz von Daten und Ausgeben eines analytischen Modells der räumlichen Korrelationen;

mittels eines Zielfunktionsaufstellungsmoduls (37) Empfangen eines Zeitreihendatensatzes betreffend Gesichtsfeldmessungen für einen konkreten Patienten als Eingabe, Aufstellen einer analytischen Funktion, die eine Wahrscheinlichkeit einer Änderung in dem Zeitreihendatensatz darstellt, aus dem Fehlermodell und dem Modell räumlicher Korrelationen;

mittels eines Optimierungsmoduls (39) Bestimmen einer Anpassung für die aufgestellte analytische Funktion an den Zeitreihendatensatz und Ausgeben eines Änderungsmodells für den analysierten Zeitreihendatensatz; und

mittels eines Berichtsmoduls (41) Generieren eines Berichts betreffend Änderungen der Netzhautfunktion in dem analysierten Datensatz aus dem Änderungsmodell.

**14.** Verfahren nach Anspruch 13, wobei einer oder mehrere der Verfahrensschritte an einer ersten Stelle vollbracht werden, die von einer zweiten Stelle entfernt ist, an der der Zeitreihendatensatz erstellt worden ist.

**15.** Computerprogramm, umfassend Anweisungen, um zu bewirken, dass das System nach Anspruch 1 die Schritte des Verfahrens nach Anspruch 13 oder 14 implementiert.

**Revendications**

**1.** Système d'analyse de changement (29) pour un ensemble de données de série temporelle concernant la structure et/ou la fonction rétinienne, le système comprenant :

un module de profilage d'erreurs (31) configuré pour recevoir un ensemble historique de données (43) en tant qu'entrée, pour déterminer les erreurs dans ledit ensemble historique de données, ledit ensemble historique de données comprenant une pluralité de mesures de champ visuel prises au niveau d'emplacements de test rétinien répartis dans l'espace sur une période de temps, et pour produire un modèle analytique desdites erreurs ;

un module de quantification de corrélation spatiale (33) configuré pour recevoir ledit ensemble historique de données en tant qu'entrée, pour déterminer une corrélation spatiale entre les emplacements de test rétinien dans ledit ensemble historique de données, et pour produire un modèle analytique desdites corrélations spatiales ;

un module de changements attendus (35) configuré pour générer un modèle analytique des changements attendus, ledit modèle comprenant une attente de changements dans un ensemble de données de série temporelle concernant le champ visuel pour un patient particulier qui doit faire l'objet d'une analyse par ledit système d'analyse de changements ;

un module de construction de fonction cible (37) configuré pour recevoir en tant qu'entrée un ensemble de données de série temporelle concernant les mesures de champ visuel pour un patient particulier, pour construire à partir dudit modèle d'erreur, et d'un modèle de corrélation spatiale, une fonction analytique représentant une probabilité de changement dans ledit ensemble de données de série temporelle ;

un module d'optimisation (39) configuré pour déterminer un ajustement pour ladite fonction analytique construite audit ensemble de données de série temporelle, et pour produire un modèle de changement pour l'ensemble de données de série temporelle analysé ; et

un module de rapport (41) configuré pour générer à partir dudit modèle de changement un rapport concernant les changements de la fonction rétinienne dans ledit ensemble de données analysé.

**2.** Système selon la revendication 1, ledit module de rapport servant à générer un rapport concernant le taux de changement dans ledit ensemble de données analysé, et/ou pour générer un rapport concernant une probabilité de changement dans ledit ensemble de données analysé, ladite probabilité de changement comprenant une probabilité de changement ayant déjà eu lieu ou une probabilité qu'un changement ait lieu à un certain moment dans le futur.

**3.** Système selon l'une quelconque des revendications précédentes, comprenant en outre un module de changements attendus configuré pour générer un modèle analytique de changements attendus, ledit modèle comprenant une attente de changements dans un ensemble de données de série temporelle qui doit être analysé par ledit système d'analyse de changements ; ledit module de construction de fonction cible étant configuré pour construire à partir dudit modèle d'erreur, dudit modèle de changements attendus et dudit modèle de corrélation spatiale, une fonction analytique représentant une probabilité de changement dans ledit ensemble de données de série temporelle.

**4.** Système selon l'une quelconque des revendications précédentes, ledit module de changements attendus étant configuré pour générer ledit modèle de changements attendus à partir de changements dans un autre ensemble de données qui est associé à l'ensemble de données à analyser telles que des données appartenant à un type de test différent d'un test ou de tests dont ledit ensemble de données à analyser est issu.

**5.** Système selon la revendication 4, l'un parmi ledit ensemble de données à analyser et ledit autre ensemble de données comprenant des données provenant d'un ou plusieurs tests fonctionnels qui mesurent la fonction d'un objet, et l'autre parmi ledit ensemble de données à analyser et ledit autre ensemble de données comprenant des données provenant d'un ou plusieurs tests structurels qui mesurent la structure d'un objet.

**6.** Système selon l'une quelconque des revendications précédentes, ledit module de profilage d'erreurs servant à modéliser une erreur hétéroscédastique et/ou non normale.

**7.** Système selon la revendication 6, ledit module de profilage d'erreurs servant à ajuster une ou un mélange de distributions de Weibull audit ensemble de données historiques.

**8.** Système selon l'une quelconque des revendications précédentes, lesdites erreurs comprenant la variabilité des mesures d'un objet mesuré au fil du temps et/ou le bruit, ledit bruit comprenant des différences entre les mesures réelles et les mesures enregistrées.

**9.** Système selon l'une quelconque des revendications précédentes, ledit module de quantification de corrélation spatiale pouvant être configuré pour un état particulier, tel qu'un glaucome ou une dégénérescence maculaire.

**10.** Système selon l'une quelconque des revendications précédentes, ledit ensemble de données de série temporelle qui doit être analysé comprenant un ensemble de données délivrées par un dispositif de test.

**11.** Système selon l'une quelconque des revendications précédentes, ledit module de construction de fonction cible servant à mettre en oeuvre un cadre bayésien, et/ou ledit module de changements attendus produisant des distributions de probabilité antérieures dans un cadre bayésien.

**12.** Dispositif de test, tel qu'un périmètre de champ visuel ou un dispositif d'imagerie de structure rétinienne, comprenant un système selon l'une quelconque des revendications précédentes.

**13.** Procédé d'analyse de changement pour un ensemble de données de série temporelle concernant la structure et/ou la fonction rétinienne, le procédé comprenant :

au moyen d'un module de profilage d'erreurs (31), la réception de données historiques en tant qu'entrée, la détermination des erreurs dans ledit ensemble historique de données ; ledit ensemble historique de données comprenant une pluralité de mesures de champ visuel prises au niveau d'emplacements de test rétinien répartis dans l'espace sur une période de temps, et la production d'un modèle analytique desdites erreurs ;
au moyen d'un module de quantification de corrélation spatiale (33), la réception en tant qu'entrée dudit ensemble historique de données, la détermination d'une corrélation spatiale entre les emplacements de test dans ledit ensemble historique de données et la production d'un modèle analytique desdites corrélations spatiales ;
au moyen d'un module de construction de fonction cible (37), la réception en tant qu'entrée d'un ensemble de données de série temporelle concernant les mesures de champ visuel pour un patient particulier, la construction à partir dudit modèle d'erreur et dudit modèle de corrélation spatiale, d'une fonction d'analyse représentant une probabilité de changement dans ledit ensemble de données de série temporelle ;
au moyen d'un module d'optimisation (39), la détermination d'un ajustement pour ladite fonction analytique construite audit ensemble de données de série temporelle, et la production d'un modèle de changement pour l'ensemble de données de série temporelle analysé ; et
au moyen d'un module de rapport (41), la génération à partir dudit modèle de changement d'un rapport concernant les changements de la fonction rétinienne dans ledit ensemble de données analysé.

14. Procédé selon la revendication 13, une ou plusieurs des étapes de procédé étant accomplies au niveau d'un premier emplacement distant d'un second emplacement où ledit ensemble de données de série temporelle a été créé.

15. Programme informatique comprenant des instructions permettant d'amener le système selon la revendication 1 à mettre en oeuvre les étapes du procédé selon les revendications 13 ou 14.

1

| PSU | SYSTEM BUS | Ethernet Interface |
| Processor | | Wireless Interface |
| Memory | | Peripheral Interface |
| Data Store | | Video Controller |

3

5

9

11

13

15

17

19

7

**_Fig. 1_**

| CHANGE ANALYSIS SYSTEM | 29 |
| APPLICATION SOFTWARE | 27 |
| OPERATING SYSTEM | 25 |
| BIOS | 21 |
| HARDWARE | 23 |

**_Fig. 2_**

**EXPECTED CHANGE MODULE** — 35

**TARGET FUNCTION CONSTRUCTION MODULE** — 37

**OPTIMISATION MODULE** — 39

**REPORTING MODULE** — 41

**ERROR PROFILING MODULE** — 31

**SPATIAL CORRELATION QUANTIFICATION MODULE** — 33

— 29

**HISTORICAL DATA** — 43

**TIME SERIES DATASET** — 45

_**Fig. 3**_

Contrast stimulus

(a)

(d) Probability of seen / Stimulus contrast (dB)

Intrathreshold — DLS — Suprathreshold

Primary visual cortex — Retina — SAP

(b)

Optic nerve head

Superior

Inferior

(c) Nasal — Temporal

(e) DLS (dB)

(f) Time

*Fig. 4*

*Fig. 5*

Fig. 6

*Fig. 7*

*Fig. 8*

*Fig. 9*

*Fig. 10*

*Fig. 11*

(a) 67.6    68.3    68.6    69.1    69.4    69.8*

(b)    Slope=0.58 dB/year
       p=9.3%

(c)    PLR

(d)    ANSWERS

**Fig. 12**

(a) 53.3    53.6    53.9    54.8*    55.2*    60.3*

(b)

Slope=-2.74 dB/year
p=13.3%

(c) ANSWER

(d) ANSWERS

Fig. 13

*Fig. 14*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DAVID P. CRABB et al.** Improving the Prediction of Visual Field Progression in Glaucoma Using Spatial Processing. *OPHTHALMOLOGY,* 01 March 1997, vol. 104 (3), 517-524